# EUROPÄISCHE PATENTSCHRIFT

(11) **EP 3 161 116 B1**
(45) Veröffentlichungstag und Bekanntmachung des Hinweises auf die Patenterteilung: **11.04.2018**
(21) Anmeldenummer: 15730145.8
(22) Anmeldetag: 18.06.2015
(51) Int. Cl.: C11D 3/386

(54) **REINIGUNGSMITTEL ENTHALTEND PROTEASEN**
CLEANING AGENT CONTAINING PROTEASES
PRODUIT DÉTERGENT CONTENANT DES PROTÉASES

(30) Priorität: 30.06.2014 DE 102014212639
(43) Veröffentlichungstag der Anmeldung: 03.05.2017
(73) Patentinhaber: Henkel AG & Co. KGaA, 40589 Düsseldorf (DE)
(72) Erfinder: EITING, Thomas, 40589 Düsseldorf (DE); MUSSMANN, Nina, 47877 Willich (DE); BASTIGKEIT, Thorsten, 42279 Wuppertal (DE); BENDA, Konstantin, 40822 Mettmann (DE); O'CONNELL, Timothy, 40547 Düsseldorf (DE); HELLMUTH, Hendrik, 40237 Düsseldorf (DE); MENKE, Silke, 42781 Haan (DE)
(86) Internationale Anmeldenummer: PCT/EP2015/063665
(87) Internationale Veröffentlichungsnummer: WO 2016/000970

(56) Entgegenhaltungen:
- EP-A1- 2 100 949
- EP-A2- 1 921 148
- WO-A1-2011/036263
- US-B1- 6 287 841

## Beschreibung

Die vorliegende Anmeldung richtet sich auf ein Reinigungsmittel, bevorzugt ein Geschirrspülmittel, vorzugsweise ein maschinelles Geschirrspülmittel, das mindestens zwei Proteasen enthält, sowie die Verwendung eines solchen Reinigungsmittels.

Das wichtigste Kriterium beim Reinigen von Textilien, harten Oberflächen, wie insbesondere beim Geschirrspülen, insbesondere beim maschinellen Geschirrspülen, ist die Reinigungsleistung an verschiedensten Anschmutzungen, welche insbesondere in Form von Lebensmittelresten eingebracht werden. Auch wenn die Reinigungsleistung der heutzutage eingesetzten Geschirrspülmittel generell hoch ist, stellt sich, auch aufgrund des generellen Trends beim maschinellen Geschirrspülen vermehrt Niedrigtemperatur-Programme zu verwenden, allerdings das Problem, dass viele der üblichen maschinellen Geschirrspülmittel bei hartnäckigen, angebrannten Anschmutzungen eine unzureichende Reinigungsleistung aufweisen. Eine solche unzureichende Reinigungsleistung und die damit einhergehende unzureichende Reinigung des Geschirrs führen beim Verbraucher zu Unzufriedenheit und dazu, dass hartnäckige Anschmutzungen vom Verbraucher vorbehandelt werden, was wiederum den Wasser- und Energieverbrauch erhöht. Daher besteht ein allgemeiner Bedarf nach maschinellen Geschirrspülmitteln, die auch an hartnäckigen, insbesondere angebrannten Anschmutzungen noch eine gute Reinigungsleistung aufweisen ohne dabei die bestehende gute Reinigungsleistung an anderen Anschmutzungen zu verringern.

Die Aufgabe der vorliegenden Erfindung bestand daher darin, ein Reinigungsmittel, bevorzugt ein Geschirrspülmittel, vorzugsweise ein maschinelles Geschirrspülmittel, zur Verfügung zu stellen, das bei solchen Anschmutzungen eine gesteigerte Reinigungsleistung aufweist, ohne dass die Reinigungsleistung an anderen Anschmutzungen verringert wird.

Überraschenderweise wurde nun festgestellt, dass die Verwendung einer Kombination verschiedener Proteasen die Reinigungsleistung von entsprechenden Reinigungsmitteln, bevorzugt eines Geschirrspülmittels, vorzugsweise eines maschinellen Geschirrspülmittels an protease-sensitiven Anschmutzungen, insbesondere angebrannten Lebensmittelanschmutzungen, insbesondere an angebrannten zuckerhaltigen Lebensmittelanschmutzungen, erheblich verbessert.

In einem ersten Aspekt richtet sich die vorliegende Erfindung daher auf ein Reinigungsmittel für harte Oberflächen, insbesondere ein Geschirrspülmittel, vorzugsweise ein maschinelles Geschirrspülmittel, das eine erste und eine zweite Protease umfasst, wobei
a) die erste Protease eine Aminosäuresequenz umfasst, die zu der in SEQ ID NO. 1 angegebenen Aminosäuresequenz über deren Gesamtlänge zu mindestens 80% identisch ist und mindestens zwei, bevorzugt mindestens drei, besonders bevorzugt vier und am meisten bevorzugt fünf der Aminosäuresubstitutionen ausgewählt aus S9R, A15T, V66A, N212D und Q239R in der Zählung gemäß SEQ ID NO. 1 aufweist, und
b) die zweite Protease eine Aminosäuresequenz umfasst, die zu der in SEQ ID NO. 2 angegebenen Aminosäuresequenz über deren Gesamtlänge zu mindestens 80 % identisch ist und mindestens eine Aminosäuresubstitution an einer, zwei, drei oder vier der folgenden Positionen 116, 126, 127, 128 und 160 in der Zählung gemäß SEQ ID NO. 2 aufweist.

Eine solche Kombination mehrerer Proteasen bewirkt bei Anwendung des Mittels eine signifikant gesteigerte Reinigungsleistung an hartnäckigen Anschmutzungen, insbesondere an eingebrannten Anschmutzungen, insbesondere an eingebrannter Milch und/oder eingebrannten Zuckerhaltigen Anschmutzungen, beispielsweise Creme Brûlée.

Ein weiterer Gegenstand der vorliegenden Erfindung ist die Verwendung eines hierin beschriebenen Reinigungsmittels, bevorzugt eines Geschirrspülmittels, vorzugsweise eines maschinellen Geschirrspülmittels in einem Reinigungsverfahren, bevorzugt einem Geschirrspülverfahren, insbesondere in einem maschinellen Geschirrspülverfahren, vorzugsweise die Verwendung zur Verbesserung der Reinigungsleistung, insbesondere der Reinigungsleistung an protease-sensitiven Anschmutzungen, an harten Oberflächen, insbesondere Geschirr bei dessen Reinigung, bevorzugt in einer automatischen Geschirrspülmaschine, insbesondere angebrannten, hartnäckigen Anschmutzungen, u.a. auch bei Temperaturen, die niedriger sind als die üblicherweise verwendeten Temperaturen.

Ein weiterer Gegenstand der vorliegenden Erfindung ist ein Reinigungsverfahren, bevorzugt Geschirrspülverfahren, vorzugsweise ein maschinelles Geschirrspülverfahren, bei dem ein hierin beschriebenes Reinigungsmittel, bevorzugt ein Geschirrspülmittel, vorzugsweise ein maschinelles Geschirrspülmittel, insbesondere zu dem Zweck, die Reinigungsleistung an angebrannten, protease-sensitiven Anschmutzungen zu verbessern, zum Einsatz kommt. In verschiedenen Ausführungsformen der Erfindung werden bei dem Geschirrspülverfahren Temperaturen eingesetzt, die niedriger sind als die üblicherweise verwendeten Temperaturen.

"Niedrige Temperaturen" oder "Temperaturen, die niedriger sind als die üblicherweise verwendeten Temperaturen", wie hierin im Zusammenhang mit Geschirrspülverfahren verwendet, bezieht sich vorzugsweise auf Temperaturen unter 60 °C, insbesondere unter 55 °C, noch bevorzugter 50 °C oder niedriger, besonders bevorzugt 45 °C oder niedriger und am bevorzugtesten 40°C oder niedriger. Diese Temperaturangaben beziehen sich auf die in den Spülschritten eingesetzten Temperaturen.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist. Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Die eingesetzten Proteasen sind alkalische Serin-Proteasen. Sie wirken als unspezifische Endopeptidasen, das heißt, sie hydrolysieren beliebige Säureamidbindungen, die im Inneren von Peptiden oder Proteinen liegen und bewirken dadurch den Abbau proteinhaltiger Anschmutzungen auf dem Reinigungsgut. Ihr pH-Optimum liegt meist im deutlich alkalischen Bereich.

Die Sequenzen der reifen (maturen) Protease Subtilisin 309 aus Bacillus lentus, welche unter dem Handelsnamen Savinase® von der Firma Novozymes A/S, Bagsvaerd, Dänemark vertrieben wird bzw. der reifen (maturen) Protease aus Bacillus alkalophilus PB92(wildtyp) sind in SEQ ID NO. 1 bzw. SEQ ID NO:2 angegeben.

"Verschieden", wie hierin mit Bezug auf die Proteasen verwendet, bezieht sich auf Proteasen, die sich in ihrer Aminosäuresequenz unterscheiden. In verschiedenen Ausführungsformen stammen voneinander verschiedene Proteasen aus unterschiedlichen Arten von Organismen oder unterscheiden sich voneinander durch, beispielsweise künstlich erzeugte, Mutationen.

"Variante", wie hierin in Zusammenhang mit Proteasen verwendet, bezieht sich auf natürliche oder artifiziell erzeugte Variationen einer nativen Protease, die eine gegenüber der Referenzform abgewandelte Aminosäuresequenz aufweisen. Eine solche Variante kann einzelne oder mehrere Punktmutationen, d.h. Substitutionen einer natürlicherweise an der entsprechenden Position vorkommenden Aminosäure durch eine andere, Insertionen (Einfügen von einer oder mehreren Aminosäuren) und/oder Deletionen (Entfernen von einer oder mehreren Aminosäuren) aufweisen, insbesondere eine oder mehrere Punktmutationen. Derartige Varianten haben vorzugsweise mindestens 50, vorzugsweise 60 oder mehr, noch bevorzugter 70, 80, 90, 100 % oder mehr der Enzymaktivität der Referenzform. In verschiedenen Ausführungsformen hat eine derartige Variante eine Aminosäuresequenz, die zu der als Referenz dienenden Sequenz über deren Gesamtlänge zu mindestens 70, vorzugsweise 75, 80, 85, 90, 95, 96, 97, 98, oder 99 % identisch ist. Die Varianten haben vorzugsweise dieselbe Länge wie die Referenzsequenz. Varianten können sich gegenüber der Referenzform durch verbesserte Eigenschaften auszeichnen, wie beispielsweise höhere Enzymaktivität, höhere Stabilität, geänderte Substratspezifität, etc.. Eingesetzt werden nur solche Varianten, die Proteaseaktivität aufweisen.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Als erste Protease im Sinne der vorliegenden Erfindung wird eine Protease eingesetzt, die eine Aminosäuresequenz umfasst, die zu der in SEQ ID NO. 1 angegebenen Aminosäuresequenz über deren Gesamtlänge zu mindestens 80% identisch ist und mindestens zwei, bevorzugt mindestens drei, besonders bevorzugt vier und am meisten bevorzugt fünf der Aminosäuresubstitutionen ausgewählt aus S9R, A15T, V66A, N212D und Q239R in der Zählung gemäß SEQ ID NO. 1 aufweist.

Weitere einsetzbare Varianten sind solche, die eine Aminosäuresequenz besitzen, die zu der in SEQ ID NO. 3 angegebenen Aminosäuresequenz mindestens zu 80%, und zunehmend bevorzugt zu mindestens 81%, 82%, 83%, 84%, 85%, 86%, 87%, 88%, 89%, 90%, 91%, 92%, 93%, 94%, 95%, 96%, 97%, 98% oder 99% identisch ist, wobei die Positionen 9, 15, 66, 212 und 239 invariabel sind, d.h. die Aminosäure an diesen Positionen der entsprechenden Aminosäure in SEQ ID NO:3 entspricht.

Besonders bevorzugt verwendet wird somit eine Variante mit mindestens einer, bevorzugt zwei, insbesondere drei, besonders bevorzugt vier oder ganz besonders bevorzugt 5 der Aminosäuresubstitutionen ausgewählt aus S9R, A15T, V66A, N212D und Q239R bezogen auf die Zählung nach SEQ ID NO:1. Bevorzugt sind die folgenden Kombinationen:
S9R+V66A+N212D+Q239R,S9R+A15T+N212D+Q239R,S9R+A15T+V66A+Q239R, S9R+A15T+V66A+N212D,A15T+V66A+N212D+Q239R;S9R+A15T+V66A,S9R+A15T+N212D, S9R+A15T+Q239R, S9R+N212D+Q239R, S9R+V66A+N212D, S9R +V66A+ Q239R, A15T+V66A+N212D,A15T+V66A+Q239R,A15T+N212D+Q239R,V66A+N212D+Q239R; S9R+A15T, S9R+V66A, S9R+N212D, S9R+ Q239R, A15T+V66A, A15T+N212D, A15T+Q239R, V66A+N212D, V66A+Q239R, N212D+Q239R. Eine Variante, die alle vorstehend genannten Änderungen umfasst, hat die in SEQ ID NO:3 angegebene Aminosäuresequenz (S9R+A15T+V66A+N212D+Q239R).

Die zweite Protease ist von der ersten Protease verschieden, d. h. eine Protease, die sowohl unter die Definition für die erste Protease als auch für die der zweiten Protease fällt, kann nicht als erste und als zweite Protease gleichzeitig gezählt werden.

Die zweite Protease umfasst erfindungsgemäß eine Aminosäuresequenz, die zu der in SEQ ID NO. 2 angegebenen Aminosäuresequenz über deren Gesamtlänge zu mindestens 80% identisch ist und mindestens eine Aminosäuresubstitution an einer, zwei, drei oder vier der folgenden Positionen 116, 126, 127, 128 und 160 in der Zählung gemäß SEQ ID NO. 2 aufweist.

Weitere einsetzbare Varianten sind solche, die eine Aminosäuresequenz besitzen, die zu der in SEQ ID NO. 2 angegebenen Aminosäuresequenz zu mindestens 80% und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist und in der Zählung gemäß SEQ ID NO. 2 an mindestens eine der folgenden Positionen 32, 33, 48-54, 58-62, 94-107, 116, 123-133, 150, 152-156, 158-161, 164, 169, 175-186, 197, 198, 203-216 eine Aminosäuresubstitution trägt. Somit wird bevorzugt als zweite Protease eine Variante der Protease mit der in SEQ ID NO:2 angegebenen Aminosäuresequenz eingesetzt, die zu der in SEQ ID NO. 2 angegebenen Aminosäuresequenz über deren Gesamtlänge zu mindestens 80% identisch ist und die mindestens eine Aminosäuresubstitution an einer der Positionen 116, 126, 127, 128 und 160 in der Zählung gemäß SEQ ID NO. 2 aufweist. Bevorzugt werden Proteasen eingesetzt, die an zwei, bevorzugt drei oder mehr, insbesondere vier der vorstehend genannten Positionen eine Aminosäuresubstitution aufweisen.

Weitere einsetzbare Varianten sind solche, die eine Aminosäuresequenz besitzen, die zu der in SEQ ID NO. 2 angegebenen Aminosäuresequenz mindestens zu 81%, 82%, 83%, 84%, 85%, 86%, 87%, 88%, 89%, 90%, 91%, 92%, 93%, 94%, 95%, 96%, 97%, 98% oder 99% identisch ist, und die mindestens eine Aminosäuresubstitution an einer der folgenden Positionen 116, 126, 127, 128 und 160 aufweisen.

Besonders bevorzugt weist die zweite Protease eine Aminosäuresequenz auf, die zu der in SEQ ID NO. 2 angegebenen Aminosäuresequenz über deren Gesamtlänge zu mindestens 80% identisch ist und die in der Zählung gemäß SEQ ID NO. 2 eine der folgenden Aminosäuresubstitutionen aufweist:
(i) G116V + S126L + P127Q + S128A
(ii) G116V + S126N + P127S + S128A + S160D
(iii) G116V + S126L + P127Q + S128A + S160D
(iv) G116V + S126V + P127E + S128K
(v) G116V + S126V + P127M + A160D
(vi) S128T
(vii) G116V + S126F + P127L + S128T
(viii) G116V + S126L + P127N + S128V
(ix) G116V + S126F + P127Q
(x) G116V + S126V + P127E + S128K +S160D
(xi) G116V + S126R + P127S + S128P
(xii) S126R + P127Q + S128D
(xiii) S126C + P127R + S128D; oder
(xiv) S126C + P127R + S128G

Bevorzugt weist die zweite Protease eine Aminosäuresequenz auf, die in der Zählung gemäß SEQ ID NO. 2 mindestens eine, bevorzugt mehrere, insbesondere jede der folgenden Aminosäuresubstitution G116V, S126L, P127Q und/oder S128A aufweist und an allen anderen Stellen zu der in SEQ ID NO. 2 angegebenen Aminosäuresequenz über deren Gesamtlänge zu mindestens 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% insbesondere zu 100 % identisch ist. Besonders bevorzugt ist eine Protease, die eine Aminosäuresequenz aufweist, die ausgehend von der Aminosäuresequenz mit der SEQ ID NO. 2 durch die Aminosäuresubstitutionen G116V, S126L, P127Q und S128A gemäß der Zählung nach SEQ ID NO. 2 erhältlich ist.

Bevorzugte Kombinationen von Proteasen sind insbesondere die Kombination der Protease mit der in SEQ ID NO:3 angegebenen Aminosäuresequenz mit einer Protease, die eine Aminosäuresequenz aufweist, die in der Zählung gemäß SEQ ID NO. 2 mindestens eine, bevorzugt mehrere, insbesondere jede der folgenden Aminosäuresubstitution G116V, S126L, P127Q und/oder S128A, und ansonsten die in SEQ ID NO:2 angegebenen Aminosäuresequenz aufweist.

In verschiedenen Ausführungsformen werden diese Kombinationen von Proteasen im Massenverhältnis bezogen auf Aktivprotein von 10:1 bis 1:10, vorzugsweise 5:1 bis 1:5, insbesondere von 2:1 bis 1:2, beispielsweise 2:3 bis 3:2, besonders bevorzugt zu gleichen Teilen eingesetzt.

Besonders bevorzugt wird die Protease mit der in SEQ ID NO:3 angegebenen Aminosäuresequenz mit der weiter oben beschriebenen zweiten Protease, insbesondere der, welche eine Aminosäuresequenz aufweist, die in der Zählung gemäß SEQ ID NO. 2 mindestens eine, bevorzugt mehrere, insbesondere jede der folgenden Aminosäuresubstitution G116V, S126L, P127Q und/oder S128A aufweist und an allen anderen Stellen zu der in SEQ ID NO. 2 angegebenen Aminosäuresequenz über deren Gesamtlänge zu 100 % identisch ist, in einem Masseverhältnis von 10:1 bis 1:10, vorzugsweise 5:1 bis 1:5, insbesondere von 2:1 bis 1:2, beispielsweise 2:3 bis 3:2, besonders bevorzugt zu gleichen Teilen eingesetzt. Überraschenderweise zeigen die hierin beschriebenen Kombinationen von verschiedenen Proteasen die Eigenschaft, die Leistung der Reinigungsmittels, bevorzugt des Geschirrspülmittels zu verbessern, indem sie zu einer verbesserten Reinigungsleistung an hartnäckigen, angebrannten Anschmutzungen führen. Die Steigerung der Reinigungsleistung ist auch bei niedrigen Temperaturen, d.h. Temperaturen, die niedriger sind als die üblicherweise in Geschirrspülverfahren verwendeten, wie oben definiert, zu beobachten. Das ermöglicht es, das Reinigungsverfahren, bevorzugt das automatische Geschirrspülverfahren bei niedrigeren Temperaturen durchzuführen und trotzdem eine gute Reinigungsleistung beizubehalten.

Dabei ist in der Regel unter der Verbesserung der Reinigungsleistung zu verstehen, dass bei Verwendung der hierin beschriebenen Reinigungsmittel, insbesondere der Geschirrspülmittel die Entfernung von Anschmutzungen, insbesondere angebrannten Anschmutzungen, auf harten Oberflächen, insbesondere Geschirr, bei dessen Reinigung bevorzugt in einer automatischen Geschirrspülmaschine im Vergleich zu der Verwendung von Reinigungsmitteln, bevorzugt Geschirrspülmitteln, die die hierin beschriebenen Enzymkombinationen nicht enthalten, merklich verbessert ist.

Die einzusetzenden Enzyme können ferner zusammen mit Begleitstoffen, etwa aus der Fermentation, oder mit Stabilisatoren konfektioniert sein.

Die Reinigungsmittel, insbesondere Geschirrspülmittel, enthalten jede Protease vorzugsweise in einer Menge von 1 x 10⁻⁸ bis 5 Gew.-% bezogen auf das jeweilige aktive Protein. Bevorzugt sind die Enzyme jeweils von 0,001 bis 2 Gew.-%, weiter bevorzugt von 0,005 bis 1,5 Gew.-%, noch weiter bevorzugt von 0,01 bis 1 Gew.-% und besonders bevorzugt von 0,01 bis 0,5 Gew.-% in diesen Mitteln enthalten.

In besonders bevorzugten Ausführungsformen wird in den hierin beschriebenen Mitteln die erste Protease in einer Gesamtmenge vom 0,01 bis 1 Gew.-%, vorzugsweise 0,025 bis 0,5 Gew.-% bezogen auf aktives Protein eingesetzt. In gleicher Weise wird vorzugsweise die zweite Protease in einer Gesamtmenge von 0,005 bis 0,75 Gew.-%, vorzugsweise 0,01 bis 0,5 Gew.-% bezogen auf aktives Protein eingesetzt. Als erste Protease wird bevorzugt 0,025 bis 0,5 Gew.-% der Protease mit der Sequenz von SEQ ID NO:3 und als zweite Protease 0,01 bis 0,5 Gew.-% der Protease die eine Aminosäuresequenz aufweist, die in der Zählung gemäß SEQ ID NO. 2 mindestens eine, bevorzugt mehrere, insbesondere jede der folgenden Aminosäuresubstitution G116V, S126L, P127Q und/oder S128A aufweist und an allen anderen Stellen zu der in SEQ ID NO. 2 angegebenen Aminosäuresequenz über deren Gesamtlänge zu 100 % identisch ist.
In flüssigen Formulierungen werden die Enzyme bevorzugt als Enzymflüssigformulierung(en) eingesetzt.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden. Die Bestimmung der Aktivproteinkonzentration erfolgt diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Die Proteasen können besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung ist eine Inhibierung der Proteolyse besonders bevorzugt. Die beschriebenen Mittel können zu diesem Zweck Stabilisatoren enthalten.

Reinigungsaktive Proteasen werden in der Regel nicht in Form des reinen Proteins sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Wie aus der vorherigen Ausführungen ersichtlich, bildet das Enzym-Protein nur einen Bruchteil des Gesamtgewichts üblicher Enzym-Zubereitungen. Bevorzugt eingesetzte Protease-Zubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 15 Gew.-% des Enzymproteins. Die beschriebenen Mittel enthalten derartige Enzym-Zubereitungen daher vorzugsweise in einer Menge von jeweils 0,1 bis 10 Gew.-%, vorzugsweise 0,2 bis 5 Gew.-% bezogen auf das Gesamtmittel.

Die hierin beschriebenen Reinigungsmittel, insbesondere die bevorzugten maschinellen Geschirrspülmittel können fester oder flüssiger Natur sein und insbesondere als pulverförmige Feststoffe, in nachverdichteter Teilchenform, als homogene Lösungen oder Suspensionen vorliegen. In einer weiteren bevorzugten Ausführungsform der Erfindung liegt das maschinelle Geschirrspülmittel in einer vorportionierten Form vor. In einer weiteren bevorzugten Ausführungsform der Erfindung weist das maschinelle Geschirrspülmittel mehrere räumlich voneinander getrennte Zusammensetzungen auf, wodurch es möglich ist, nicht kompatible Inhaltsstoffe voneinander zu trennen, oder Zusammensetzungen in Kombination anzubieten, welche zu unterschiedlichen Zeitpunkten in der Geschirrspülmaschine zum Einsatz kommen. Dies ist besonders vorteilhaft, wenn die maschinellen Geschirrspülmittel in vorportionierter Form vorliegen. Dabei kann mindestens eine der Zusammensetzungen fest und/oder mindestens eine der Zusammensetzungen flüssig vorliegen, wobei die Proteasen in mindestens einer der Zusammensetzungen enthalten sind, aber auch in mehreren Zusammensetzungen vorliegen können.

Vorzugsweise enthalten die hierin beschriebenen Mittel mindestens einen weiteren Bestandteil, insbesondere mindestens zwei weitere Bestandteile, ausgewählt aus der Gruppe bestehend aus Gerüststoffen, Tensiden, Polymeren, Bleichmitteln, Bleichkatalysatoren, Bleichaktivatoren, Nicht-Protease-Enzymen, Korrosionsinhibitoren und Glaskorrosionsinhibitoren, Desintegrationshilfsmitteln, Duftstoffen und Parfümträgern.

Nachfolgend werden mögliche Inhaltsstoffe beschrieben, welche vorteilhafterweise in den hierin beschriebenen Reinigungsmittel, insbesondere Geschirrspülmittel, bevorzugt maschinellen Geschirrspülmitteln eingesetzt werden können.

Vorteilhafterweise können Gerüststoffe eingesetzt werden. Zu den Gerüststoffen zählen insbesondere die Zeolithe, Silikate, Carbonate, organische Cobuilder und -wo keine ökologischen Vorurteile gegen ihren Einsatz bestehen - auch die Phosphate.

In den hierin beschriebenen Mitteln können kristalline schichtförmige Silikate eingesetzt werden. Solche Reinigungsmittel, bevorzugt Geschirrspülmittel, insbesondere maschinellen Geschirrspülmittel enthalten vorzugsweise einen Gewichtsanteil an kristallinem schichtförmigen Silikat von 0,1 bis 20 Gew.-%, bevorzugt von 0,2 bis 15 Gew.-% und insbesondere von 0,4 bis 10 Gew.-%, jeweils bezogen auf das Gesamtgewicht dieser Mittel.

Auch ein Einsatz der allgemein bekannten Phosphate als Buildersubstanzen ist möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- bzw. Pentakaliumtriphosphat (Natrium- bzw. Kaliumtripolyphosphat) in der Wasch- oder Reinigungsmittel-Industrie die größte Bedeutung.

Alkalimetallphosphate ist dabei die summarische Bezeichnung für die Alkalimetall- (insbesondere Natrium- und Kalium-) Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei.

Technisch besonders wichtige Phosphate sind das Pentanatriumtriphosphat, Na₅P₃O₁₀ (Natriumtripolyphosphat) sowie das entsprechende Kaliumsalz Pentakaliumtriphosphat, K₅P₃O₁₀ (Kaliumtripolyphosphat). Bevorzugt einsetzbar sind die Natriumkaliumtripolyphosphate.

Werden im Rahmen der vorliegenden Anmeldung Phosphate als wasch- oder reinigungsaktive Substanzen in den Reinigungsmitteln, bevorzugt Geschirrspülmitteln, insbesondere im maschinellen Geschirrspülmittel eingesetzt, so enthalten bevorzugte Mittel diese(s) Phosphat(e), vorzugsweise Alkalimetallphosphat(e), besonders bevorzugt Pentanatrium- bzw. Pentakaliumtriphosphat (Natrium- bzw. Kaliumtripolyphosphat), in Mengen von 5 bis 80 Gew.-%, vorzugsweise von 15 bis 75 Gew.-% und insbesondere von 20 bis 70 Gew.-%, jeweils bezogen auf das Gewicht des Reinigungsmittel, bevorzugt Geschirrspülmittels, insbesondere maschinellen Geschirrspülmittels.

Weitere Gerüststoffe sind die Alkaliträger. Als Alkaliträger gelten beispielsweise Alkalimetallhydroxide, Alkalimetallcarbonate, Alkalimetallhydrogencarbonate, Alkalimetallsesquicarbonate, die genannten Alkalisilikate, Alkalimetasilikate, und Mischungen der vorgenannten Stoffe, wobei im Sinne dieser Erfindung bevorzugt die Alkalicarbonate, insbesondere Natriumcarbonat, Natriumhydrogencarbonat oder Natriumsesquicarbonat eingesetzt werden können. Besonders bevorzugt ist ein Buildersystem enthaltend eine Mischung aus Tripolyphosphat und Natriumcarbonat. Ebenfalls besonders bevorzugt ist ein Buildersystem enthaltend eine Mischung aus Tripolyphosphat und Natriumcarbonat und Natriumdisilikat. Aufgrund ihrer im Vergleich mit anderen Buildersubstanzen geringen chemischen Kompatibilität mit den übrigen Inhaltsstoffen von Reinigungsmitteln, insbesondere Geschirrspülmitteln, bevorzugt maschinellen Geschirrspülmitteln, werden die optionalen Alkalimetallhydroxide bevorzugt nur in geringen Mengen oder überhaupt nicht eingesetzt.

Besonders bevorzugt ist der Einsatz von Carbonat(en) und/oder Hydrogencarbonat(en), vorzugsweise Alkalicarbonat(en), besonders bevorzugt Natriumcarbonat, in Mengen von 2 bis 50

Gew.-%, vorzugsweise von 5 bis 40 Gew.-% und insbesondere von 7,5 bis 30 Gew.-%, jeweils bezogen auf das Gewicht des Mittels, bevorzugt maschinellen Geschirrspülmittels. Besonders bevorzugt werden Mittel, welche bezogen auf das Gewicht des maschinellen Geschirrspülmittels weniger als 20 Gew.-%, vorzugsweise weniger als 17 Gew.-%, bevorzugt weniger als 13 Gew.-% und insbesondere weniger als 9 Gew.-% Carbonat(e) und/oder Hydrogencarbonat(e), vorzugsweise Alkalicarbonat(e), besonders bevorzugt Natriumcarbonat enthalten.

Als organische Cobuilder sind insbesondere Polycarboxylate / Polycarbonsäuren, polymere Polycarboxylate, Asparaginsäure, Polyacetale, Dextrine, weitere organische Cobuilder sowie Phosphonate zu nennen. Diese Stoffklassen werden nachfolgend beschrieben.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes Mittels. Insbesondere sind hierbei Citronensäure, Bernsteinsäure, Glutarsäure, Adipinsäure, Gluconsäure und beliebige Mischungen aus diesen zu nennen.

Als besonders vorteilhaft für die Reinigungs- und Klarspülleistung hierin beschriebener Mittel hat sich der Einsatz von Citronensäure und/oder Citraten in diesen Mitteln erwiesen. Bevorzugt werden daher Reinigungsmittel, bevorzugt Geschirrspülmittel, insbesondere bevorzugt maschinelle Geschirrspülmittel, dadurch gekennzeichnet, dass das Mittel Citronensäure oder ein Salz der Citronensäure enthält und das der Gewichtsanteil der Citronensäure oder des Salzes der Citronensäure vorzugsweise mehr als 10 Gew.-%, bevorzugt mehr als 15 Gew.-% und insbesondere zwischen 20 und 40 Gew.-% beträgt.

Eine weitere bedeutende Klasse der phosphatfreien Gerüststoffe stellen Aminocarbonsäuren und/oder ihre Salze dar. Besonders bevorzugte Vertreter dieser Klasse sind Methylglycindiessigsäure (MGDA) oder ihre Salze sowie Glutamindiessigsäure (GLDA) oder ihre Salze oder Ethylendiamindiessigsäure oder ihre Salze (EDDS). Der Gehalt an diesen Aminocarbonsäuren bzw. ihren Salzen kann beispielsweise zwischen 0,1 und 15 Gew.-% bevorzugt zwischen 0,5 und 10 Gew.-% und insbesondere zwischen 0,5 und 6 Gew.-% ausmachen. Aminocarbonsäuren und ihre Salze können zusammen mit den vorgenannten Gerüststoffen, insbesondere auch mit den phosphatfreien Gerüststoffen eingesetzt werden.

Als Gerüststoffe sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol. Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure.

Die (co-)polymeren Polycarboxylate können entweder als Pulver oder als wässrige Lösung eingesetzt werden. Der Gehalt der Reinigungsmittel, bevorzugt Geschirrspülmittel, insbesondere maschinellen Geschirrspülmittel an (co-)polymeren Polycarboxylaten beträgt vorzugsweise 0,5 bis 20 Gew.-% und insbesondere 3 bis 10 Gew.-%.

Zur Verbesserung der Wasserlöslichkeit können die Polymere auch Allylsulfonsäuren, wie beispielsweise Allyloxybenzolsulfonsäure und Methallylsulfonsäure, als Monomer enthalten. Weitere bevorzugte Copolymere sind solche, die als Monomere Acrolein und Acrylsäure/Acrylsäuresalze bzw. Acrolein und Vinylacetat aufweisen.

Darüber hinaus können alle Verbindungen, die in der Lage sind, Komplexe mit Erdalkaliionen auszubilden, als Gerüststoffe eingesetzt werden.

Die hierin beschriebenen Mittel können Tenside enthalten, wobei zur Gruppe der Tenside die nichtionischen, die anionischen, die kationischen und die amphoteren Tenside gezählt werden.

Als nichtionische Tenside können alle dem Fachmann bekannten nichtionischen Tenside eingesetzt werden. Als nichtionische Tenside eignen sich beispielsweise Alkylglykoside der allgemeinen Formel RO(G)ₓ, in der R einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt einsetzbarer nichtionischer Tenside, die entweder als alleiniges nichtionisches Tensid oder in Kombination mit anderen nichtionischen Tensiden eingesetzt werden können, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind die als PHFA bekannten Polyhydroxyfettsäureamide.

Als bevorzugte Tenside können schwachschäumende nichtionische Tenside eingesetzt werden. Mit besonderem Vorzug enthalten die Reinigungsmittel, bevorzugt Geschirrspülmittel, insbesondere maschinellen Geschirrspülmittel nichtionische Tenside aus der Gruppe der alkoxylierten Alkohole. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 Mol EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt einer ganzen oder einer gebrochenen Zahl entsprechen können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Insbesondere bevorzugt sind nichtionische Tenside, die einen Schmelzpunkt oberhalb Raumtemperatur aufweisen. Nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, ist/sind besonders bevorzugt.

Bevorzugt einzusetzende Tenside stammen aus den Gruppen der alkoxylierten Niotenside, insbesondere der ethoxylierten primären Alkohole.

Aniontenside können ebenfalls als Bestandteil von Reinigungsmitteln, bevorzugt Geschirrspülmitteln, insbesondere von maschinellen Geschirrspülmitteln eingesetzt werden. Zu ihnen zählen insbesondere Alkylbenzolsulfonate, (Fett-)Alkylsulfate, (Fett-)Alkylethersulfate sowie Alkansulfonate. Der Gehalt der Mittel an Aniontensiden beträgt üblicherweise 0 bis 10 Gew.-%.

An Stelle der genannten Tenside oder in Verbindung mit ihnen können auch kationische und/oder amphotere Tenside eingesetzt werden. In Reinigungsmittel, insbesondere Geschirrspülmitteln, bevorzugt maschinellen Geschirrspülmitteln, beträgt der Gehalt an kationischen und/oder amphoteren Tensiden vorzugsweise weniger als 6 Gew.-%, bevorzugt weniger als 4 Gew.-%, ganz besonders bevorzugt weniger als 2 Gew.-% und insbesondere weniger als 1 Gew.-%. Mittel, welche keine kationischen oder amphoteren Tenside enthalten, werden besonders bevorzugt.

Zur Gruppe der Polymere zählen insbesondere die wasch- oder reinigungsaktiven Poylmere, beispielsweise die Klarspülpolymere und/oder als Enthärter wirksame Polymere. Generell sind in maschinellen Geschirrspülmitteln neben nichtionischen Polymeren auch kationische, anionische und amphotere Polymere einsetzbar.

"Kationische Polymere" im Sinne der vorliegenden Erfindung sind Polymere, welche eine positive Ladung im Polymermolekül tragen. Diese kann beispielsweise durch in der Polymerkette vorliegende (Alkyl-)Ammoniumgruppierungen oder andere positiv geladene Gruppen realisiert werden. Besonders bevorzugte kationische Polymere stammen aus den Gruppen der quaternierten Cellulose-Derivate, der Polysiloxane mit quaternären Gruppen, der kationischen Guar-Derivate, der polymeren Dimethyldiallylammoniumsalze und deren Copolymere mit Estern und Amiden von Acrylsäure und Methacrylsäure, der Copolymere des Vinylpyrrolidons mit quaternierten Derivaten des Dialkylaminoacrylats und -methacrylats, der Vinylpyrrolidon-Methoimidazoliniumchlorid-Copolymere, der quaternierter Polyvinylalkohole oder der unter den INCI-Bezeichnungen Polyquaternium 2, Polyquaternium 17, Polyquaternium 18 und Polyquaternium 27 angegeben Polymere.

"Amphotere Polymere" im Sinne der vorliegenden Erfindung weisen neben einer positiv geladenen Gruppe in der Polymerkette weiterhin auch negativ geladenen Gruppen bzw. Monomereinheiten auf. Bei diesen Gruppen kann es sich z.B. um Carbonsäuren, Sulfonsäuren oder Phosphonsäuren handeln.

Bevorzugte einsetzbare amphotere Polymere stammen aus der Gruppe der Alkylacrylamid/Acrylsäure-Copolymere, der Alkylacrylamid/Methacrylsäure-Copolymere, der Alkylacrylamid/Methylmethacrylsäure-Copolymere, der Alkylacrylamid/Acrylsäure/Alkylaminoalkyl(meth)acrylsäure-Copolymere, der Alkylacrylamid/Methacrylsäure/Alkylaminoalkyl(meth)-acrylsäure-Copolymere, der Alkylacrylamid/Methylmethacrylsäure/Alkylaminoalkyl(meth)acrylsäure-Copolymere, der Alkylacrylamid/Alkymethacrylat/Alkylaminoethylmethacrylat/Alkylmethacrylat-Copolymere sowie der Copolymere aus ungesättigten Carbonsäuren, kationisch derivatisierten ungesättigten Carbonsäuren und gegebenenfalls weiteren ionischen oder nichtionogenen Monomeren.

Bevorzugt einsetzbare zwitterionische Polymere stammen aus der Gruppe der Acrylamidoalkyltrialkylammoniumchlorid/Acrylsäure-Copolymere sowie deren Alkali- und Ammoniumsalze, der Acrylamidoalkyltrialkylammoniumchlorid/Methacrylsäure-Copolymere sowie deren Alkali- und Ammoniumsalze und der Methacroylethylbetain/Methacrylat-Copolymere.

Reinigungsmittel, insbesondere Geschirrspülmittel, bevorzugt maschinelle Geschirrspülmittel enthalten die vorgenannten kationischen und/oder amphoteren Polymere vorzugsweise in Mengen zwischen 0,01 und 10 Gew.-%, jeweils bezogen auf das Gesamtgewicht des maschinellen Geschirrspülmittels. Bevorzugt werden im Rahmen der vorliegenden Anmeldung jedoch solche maschinelle Geschirrspülmittel, bei denen der Gewichtsanteil der kationischen und/oder amphoteren Polymere zwischen 0,01 und 8 Gew.-%, vorzugsweise zwischen 0,01 und 6 Gew.-%, bevorzugt zwischen 0,01 und 4 Gew.-%, besonders bevorzugt zwischen 0,01 und 2 Gew.-% und insbesondere zwischen 0,01 und 1 Gew.-%, jeweils bezogen auf das Gesamtgewicht des maschinellen Geschirrspülmittels, beträgt.

In den Reinigungsmitteln, bevorzugt Geschirrspülmitteln, insbesondere maschinellen Geschirrspülmitteln können ferner Bleichmittel eingesetzt werden. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Einsetzbar sind außerdem alle weiteren dem Fachmann aus dem Stand der Technik bekannten anorganischen oder organischen Peroxybleichmittel.

Als Bleichmittel können auch Chlor oder Brom freisetzende Substanzen eingesetzt werden. Unter den geeigneten Chlor oder Brom freisetzenden Materialien kommen beispielsweise heterozyklische N-Brom- und N-Chloramide, beispielsweise Trichlorisocyanursäure, Tribromisocyanursäure, Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

Es werden Reinigungsmittel, bevorzugt Geschirrspülmittel, insbesondere maschinelle Geschirrspülmittel bevorzugt, die 1 bis 35 Gew.-%, vorzugsweise 2,5 bis 30 Gew.-%, besonders bevorzugt 3,5 bis 20 Gew.-% und insbesondere 5 bis 15 Gew.-% Bleichmittel, vorzugsweise Natriumpercarbonat, enthalten.

Ferner können die Reinigungsmittel, bevorzugt Geschirrspülmittel, insbesondere maschinellen Geschirrspülmittel Bleichkatalysatoren enthalten. Die einsetzbaren Bleichkatalysatoren schließen ein, sind aber nicht beschränkt auf die Gruppe der bleichverstärkenden Übergangsmetallsalze und Übergangsmetallkomplexe, vorzugsweise der Mn-, Fe-, Co-, Ru - oder Mo-Komplexe, besonders bevorzugt aus der Gruppe der Mangan und/oder Cobaltsalze und/oder -komplexe, insbesondere der Cobalt(ammin)-Komplexe, der Cobalt(acetat)-Komplexe, der Cobalt(Carbonyl)-Komplexe, der Chloride des Cobalts oder Mangans, des Mangansulfats und der Komplexe des Mangans mit 1,4,7-trimethyl-1,4,7-triazacyclononan (Mn₃-TACN) oder 1,2,4,7-tetramethyl-1,4,7-triazacyclononan (Mn₄-TACN).

Es werden Reinigungsmittel, bevorzugt Geschirrspülmittel, insbesondere maschinelle Geschirrspülmittel bevorzugt, die 0,001 bis 1 Gew.-%, vorzugsweise 0,01 bis 0,1 Gew.-% Bleichkatalysator, vorzugsweise einen Mn-Komplex, insbesondere einen Komplex des Mangans mit 1,4,7-trimethyl-1,4,7-triazacyclononan (Mn₃-TACN)oder 1,2,4,7-tetramethyl-1,4,7-triazacyclononan (Mn₄-TACN), enthalten.

In verschiedenen Ausführungsformen der Erfindung enthalten die Reinigungsmittel, bevorzugt Geschirrspülmittel, insbesondere maschinellen Geschirrspülmittel zusätzlich mindestens einen Bleichaktivator. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Von allen dem Fachmann aus dem Stand der Technik bekannten Bleichaktivatoren werden mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS) besonders bevorzugt eingesetzt. Auch Kombinationen konventioneller Bleichaktivatoren können eingesetzt werden. Diese Bleichaktivatoren werden vorzugsweise in Mengen bis 10 Gew.-%, insbesondere 0,1 Gew.-% bis 8 Gew.-%, besonders 2 bis 8 Gew.-% und besonders bevorzugt 2 bis 6 Gew.-%, jeweils bezogen auf das Gesamtgewicht der bleichaktivatorhaltigen Mittel, eingesetzt.

Vorzugsweise enthalten die Mittel der vorliegenden Erfindung mindestens eine zusätzliche Enzymzubereitung oder Enzymzusammensetzung, die ein oder mehrere Nicht-Protease-Enzyme enthält. Solche Enzyme umfassen, ohne darauf beschränkt zu sein, Amylasen, Lipasen, Cellulasen, Hemicellulasen, Mannanasen, Pektin-spaltende Enzyme, Tannasen, Xylanasen, Xanthanasen, β-Glucosidasen, Carrageenasen, Perhydrolasen, Oxidasen, Oxidoreduktasen sowie deren Gemische. Bevorzugte Enzyme umfassen insbesondere Amylasen, insbesondere α-Amylasen, Cellulasen, Lipasen, Hemicellulasen, insbesondere Pectinasen, Mannanasen, β-Glucanasen, sowie deren Gemische. Besonders bevorzugt sind Amylasen und/oder Lipasen sowie deren Gemische. Diese Enzyme sind im Prinzip natürlichen Ursprungs; ausgehend von den natürlichen Molekülen stehen für den Einsatz in Wasch- oder Reinigungsmitteln verbesserte Varianten zur Verfügung, die entsprechend bevorzugt eingesetzt werden.

Die im Zusammenhang mit den eingesetzten Proteasen gemachten Angaben zu Mengen und Formulierungsformen treffen mutatis mutandis auch auf alle weiteren oben beschriebenen Enzyme zu.

Glaskorrosionsinhibitoren verhindern das Auftreten von Trübungen, Schlieren und Kratzern aber auch das Irisieren der Glasoberfläche von maschinell gereinigten Gläsern. Bevorzugte Glaskorrosionsinhibitoren stammen aus der Gruppe der Magnesium- und Zinksalze sowie der Magnesium- und Zinkkomplexe. Im Rahmen der vorliegenden Erfindung beträgt der Gehalt an Zinksalz in Reinigungsmitteln, bevorzugt Geschirrspülmitteln, insbesondere maschinellen Geschirrspülmitteln vorzugsweise zwischen 0,1 bis 5 Gew.-%, bevorzugt zwischen 0,2 bis 4 Gew.-% und insbesondere zwischen 0,4 bis 3 Gew.-%, bzw. der Gehalt an Zink in oxidierter Form (berechnet als Zn²⁺) zwischen 0,01 bis 1 Gew.-%, vorzugsweise zwischen 0,02 bis 0,5 Gew.-% und insbesondere zwischen 0,04 bis 0,2 Gew.-%, jeweils bezogen auf das Gesamtgewicht des Glaskorrosionsinhibitor-haltigen Mittels.

Um den Zerfall vorgefertigter Formkörper zu erleichtern, ist es möglich, Desintegrationshilfsmittel, so genannte Tablettensprengmittel, in diese Mittel einzuarbeiten, um die Zerfallszeiten zu verkürzen. Unter Tablettensprengmitteln bzw. Zerfallsbeschleunigern werden Hilfsstoffe verstanden, die für den raschen Zerfall von Tabletten in Wasser oder anderen Medien und für die zügige Freisetzung der Wirkstoffe sorgen. Bevorzugt können Desintegrationshilfsmittel in Mengen von 0,5 bis 10 Gew.-%, vorzugsweise 3 bis 7 Gew.-% und insbesondere 4 bis 6 Gew.-%, jeweils bezogen auf das Gesamtgewicht des desintegrationshilfsmittelhaltigen Mittels, eingesetzt werden.

Als Parfümöle bzw. Duftstoffe können im Rahmen der vorliegenden Erfindung einzelne Riechstoffverbindungen, z.B. die synthetischen Produkte vom Typ der Ester, Ether, Aldehyde, Ketone, Alkohole und Kohlenwasserstoffe verwendet werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pinien-, Citrus-, Jasmin-, Patchouli-, Rosen- oder Ylang-Ylang-Öl.

Die Konfektionierung hierin beschriebener Reinigungsmittel, bevorzugt Geschirrspülmittel, insbesondere maschineller Geschirrspülmittel kann in unterschiedlicher Wiese erfolgen. Die Mittel können in fester oder flüssiger sowie als Kombination fester und flüssiger Angebotsformen vorliegen. Als feste Angebotsformen eignen sich insbesondere Pulver, Granulate, Extrudate, Kompaktate, insbesondere Tabletten. Die flüssigen Angebotsformen auf Basis von Wasser und/oder organischen Lösungsmitteln können verdickt, in Form von Gelen vorliegen. Hierin beschriebene Mittel können in Form einphasiger oder mehrphasiger Produkte konfektioniert werden.

Hierin beschriebene Reinigungsmittel, bevorzugt Geschirrspülmittel, insbesondere maschinelle Geschirrspülmittel werden vorzugsweise zu Dosiereinheiten vorkonfektioniert. Diese Dosiereinheiten umfassen vorzugsweise die für einen Reinigungsgang notwendige Menge an wasch- oder reinigungsaktiven Substanzen.

Die hierin beschriebenen Reinigungsmittel, bevorzugt Geschirrspülmittel, insbesondere maschinellen Geschirrspülmittel, insbesondere die vorgefertigten Dosiereinheiten weisen mit besonderem Vorzug eine wasserlösliche Umhüllung auf.

Die wasserlösliche Umhüllung wird vorzugsweise aus einem wasserlöslichen Folienmaterial, welches ausgewählt ist aus der Gruppe, bestehend aus Polymeren oder Polymergemischen, gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein. Besonders bevorzugt sind Folien, die beispielsweise zu Verpackungen wie Schläuchen oder Kissen verklebt und/oder versiegelt werden können, nachdem sie mit einem Mittel befüllt wurden.

Es ist bevorzugt, dass die wasserlösliche Umhüllung Polyvinylalkohol oder ein Polyvinylalkoholcopolymer enthält. Wasserlösliche Umhüllungen, die Polyvinylalkohol oder ein Polyvinylalkoholcopolymer enthalten, weisen eine gute Stabilität bei einer ausreichend hohen Wasserlöslichkeit, insbesondere Kaltwasserlöslichkeit, auf.

Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10.000 bis 1.000.000 gmol⁻¹, vorzugsweise von 20.000 bis 500.000 gmol⁻¹, besonders bevorzugt von 30.000 bis 100.000 gmol⁻¹ und insbesondere von 40.000 bis 80.000 gmol⁻¹ liegt.

Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% ausmacht.

Einem zur Herstellung der wasserlöslichen Umhüllung geeignetem Polyvinylalkohol-enthaltendem Folienmaterial kann zusätzlich ein Polymer ausgewählt aus der Gruppe umfassend (Meth)Acrylsäure-haltige (Co)Polymere, Polyacrylamide, Oxazolin-Polymere, Polystyrolsulfonate, Polyurethane, Polyester, Polyether, Polymilchsäure oder Mischungen der vorstehenden Polymere zugesetzt sein. Ein bevorzugtes zusätzliches Polymer sind Polymilchsäuren.

Bevorzugte Polyvinylalkoholcopolymere umfassen neben Vinylalkohol Dicarbonsäuren als weitere Monomere. Geeignete Dicarbonsäuren sind Itaconsäure, Malonsäure, Bernsteinsäure und Mischungen daraus, wobei Itaconsäure bevorzugt ist.

Ebenfalls bevorzugte Polyvinylalkoholcopolymere umfassen neben Vinylalkohol eine ethylenisch ungesättige Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Es kann bevorzugt sein, dass das Folienmaterial weitere Zusatzstoffe enthält. Das Folienmaterial kann beispielsweise Weichmacher wie Dipropylenglycol, Ethylenglycol, Diethylenglycol, Propylenglycol, Glycerin, Sorbitol, Mannitol oder Mischungen daraus enthalten. Weitere Zusatzstoffe umfassen beispielsweise Freisetzungshilfen, Füllmittel, Vernetzungsmittel, Tenside, Antioxidationsmittel, UV-Absorber, Antiblockmittel, Antiklebemittel oder Mischungen daraus.

Geeignete wasserlösliche Folien zum Einsatz in den wasserlöslichen Umhüllungen der wasserlöslichen Verpackungen gemäß der Erfindung sind Folien, die von der Firma MonoSol LLC beispielsweise unter der Bezeichnung M8630, C8400 oder M8900 vertrieben werden. Andere geeignete Folien umfassen Folien mit der Bezeichnung Solublon® PT, Solublon® GA, Solublon® KC oder Solublon® KL von der Aicello Chemical Europe GmbH oder die Folien VF-HP von Kuraray.

Die entsprechende Verwendung der hierin beschriebenen Mittel ist ebenfalls Gegenstand der Erfindung. Ebenso betrifft die Erfindung ein Geschirrspülverfahren, insbesondere maschinelles Geschirrspülverfahren, bei welchem ein Mittel gemäß der Erfindung eingesetzt wird. Gegenstand der vorliegenden Anmeldung ist daher weiterhin ein Verfahren zur Reinigung von Geschirr in einer Geschirrspülmaschine, bei welchem das Mittel während des Durchlaufens eines Geschirrspülprogramms vor Beginn des Hauptspülgangs oder im Verlaufe des Hauptspülgangs in den Innenraum einer Geschirrspülmaschine eindosiert wird. Die Eindosierung bzw. der Eintrag des Mittels in den Innenraum der Geschirrspülmaschine kann manuell erfolgen, vorzugsweise wird das Mittel jedoch mittels der Dosierkammer in den Innenraum der Geschirrspülmaschine dosiert. In verschiedenen Ausführungsformen der Erfindung liegt bei solchen Geschirrspülverfahren die (Spül-)Temperatur vorzugsweise bei 50 °C oder niedriger, besonders bevorzugt 45 °C oder niedriger, noch bevorzugter 40°C oder niedriger.

Eine typische Rahmenrezeptur für ein vorzugsweise einsetzbares maschinelles Geschirrspülmittel, beispielsweise in Tablettenform, umfasst folgende Stoffe:

| | |
|---|---|
| Na-Tripolyphosphat | 20-50 Gew.-% |
| Natriumcarbonat | 10-30 Gew.-% |
| Natriumpercarbonat | 5-18 Gew.-% |
| Bleichaktivator | 0,5-5 Gew.-% |
| Bleichkatalysator | 0,01-1 Gew.-% |
| Sulfopolymer | 2,5-15 Gew.-% |
| Polycarboxylat | 0,1-10 Gew.-% |
| Niotensid | 0,5-10 Gew.-% |
| Phosphonat | 0,5-5 Gew.-% |
| Proteasen | 0,1-5 Gew.-% |
| Amylase | 0,1-5 Gew.-%, |

wobei sich die Angabe in Gew.-% jeweils auf das gesamte Mittel beziehen. Statt des oder eines Teils des Tripolyphosphats kann in der Rezeptur insbesondere auch 10-50 Gew.-% Citrat oder MGDA oder GLDA oder EDDS oder Mischungen aus zwei oder drei dieser Substanzen eingesetzt werden.

### Beispiele

### Beispiel 1: Verbesserung der Proteaseleistung beim automatischen Geschirrspülen

**Tabelle 1: Zusammensetzung des maschinellen Geschirrspülmittels**

| | Basis |
|---|---|
| Phosphat (Gew.-%) | 35,9 |
| Natriumcarbonat (Gew.-%) | 12,2 |
| Phosphonat (Gew.-%) | 2,4 |
| Sulfonsäuregruppen-haltiges Polymer (Gew.-%) | 7,9 |
| Polyacrylat (Gew.-%) | 4,6 |
| Nichtionische Tenside (Gew.-%) | 6,1 |
| Percarbonat (Gew.-%) | 14,6 |
| TAED (Gew.-%) | 2,3 |
| Bleichkatalysator (Gew.-%) | 1,0 |
| Polycarboxylat (Gew.-%) | 1,5 |
| Natriumsilikat/Polycarboxylat (Gew.%) | 3,9 |
| Enzymzusammensetzung (Amylase) (Gew.-%) | 1,0 |
| Zinkacetat (Gew.-%) | 0,2 |
| Reste (Parfüm, Farbstoffe, Protease bzw. Proteasemischung etc.) (Gew.-%) | ad 100 |

### Beispiel 1: Reinigungsleistung an Creme Brûlée

In einer Miele G698 Geschirrspülmaschine wurden bei 50 °C (Programm "Normal") und 21°dH Porzellanteller mit einer Anschmutzung aus schwarzem Tee, Eigelb, Hackfleisch und Creme Brûlée mit einer festen Geschirrspülmitteltablette (20 g; Zusammensetzung siehe Tabelle 1) mit verschiedenen einzelnen Proteasen (Vergleichsversuche V1, V2) oder Proteasekombinationen (M1) gespült. Die Creme Brûlée Anschmutzung wurde als hartnäckige Anschmutzung verwendet. Dazu wurde die fertige Mischung Creme Brûlée der Firma Debic in einem Topf auf 60°C erwärmt und jeweils 3,5 g mit einem Pinsel auf Dessertteller aufgetragen und für 2 h bei Raumtemperatur eintrocknen gelassen. Danach wurden die Teller in einen kalten Ofen (Binder) gestellt und innerhalb von 1 h auf 140°C erhitzt. Dann wurde die Creme Brûlée Anschmutzung für 2 h bei 140°C im Ofen eingebrannt.

Nach jedem Spülzyklus wurde die Reinigungsleistung visuell nach IKW bestimmt (Auswertung von 1-10, je höher der Wert, desto besser die Leistung, Unterschiede von mindestens 1 sind signifikant). Die Ergebnisse für die gestesteten Rezepturen sind in der Tabelle 2 als arithmetische Mittelwerte aufgelistet. Höhere Werte bedeuten eine bessere Reinigungsleistung.

Zur Formulierung gemäß Tabelle 1 wurde soviel der entsprechenden Proteasezubereitung (einzelne Proteasen, V1 und V2) bzw. deren Mischungen (M1, Masseverhältnis der Protease V1:V2, 2:3) zugegeben, dass die Gesamtproteasemenge in der Formulierung 0,24 Gew.-% betrug.

**Tabelle 2: Reinigungsleistung an Creme Brulée**

| Protease/Proteasekombination | Tee | Eigelb | Stärke | Creme Brûlée |
|---|---|---|---|---|
| M1: | 10,0 | 9,5 | 9,3 | 8,6 |
| V1: | 9,5 | 8,7 | 8,7 | 6,2 |
| V2: | 6,0 | 9,6 | 9,7 | 7,6 |

| | | | | |
|---|---|---|---|---|
| V1: Basis + 1.Protease: (Enzym gemäß Seq ID NO. 3) V2: Basis + 2. Protease (Protease, die eine Aminosäuresequenz aufweist, die in der Zählung gemäß SEQ ID NO. 2 die folgenden Aminosäuresubstitution G116V, S126L, P127Q und S128A, und ansonsten die in SEQ ID NO:2 angegebenen Aminosäuresequenz aufweist) M1: Basis + Mischung im Verhältnis 2:3 bezogen auf den Proteasegehalt in Gew.-% aus Enzym gemäß V1 und V2 | | | | |

Wie man der Tabelle 2 entnehmen kann zeigt die erste Protease (V1) eine sehr gute Reinigungsleistung an Teeanschmutzungen, während die Reinigungsleistung an Creme Brûlée nur mittelmäßig ist. Dagegen erkennt man bei der zweiten Protease (V2) eine gute Leistung an Creme Brûlée, während die Leistung an Tee nur mittelmäßig ist.

Der Tabelle 2 ist eindeutig zu entnehmen, dass die Kombination der zwei verschiedenen Proteasen zu einer deutlichen Verbesserung der Reinigungsleistung führt (M1).

### Beispiel 2: Reinigungsleistung an Milch

In einer Bosch SMS86 Geschirrspülmaschine wurden bei 40 °C (Programm "Sanft 40") und 21 °dH Porzellanteller mit einer Anschmutzung aus Milch mit einer festen Geschirrspülmitteltablette gespült. Zur Formulierung gemäß Tabelle 1 wurde soviel der entsprechenden Proteasezubereitung (einzelne Proteasen, V1 und V2) bzw. deren Mischungen (M1, Masseverhältnis der Protease V1:V2, 5:1) zugegeben, dass die Gesamtproteasemenge in der Formulierung 0,24 Gew.-% betrug. Die Milch Anschmutzung wurde durch Einbrennen von Milch mit Fettgehalt von 1,5 % in der Mikrowelle erzeugt.

**Tabelle 3: Reinigungsleistung an Milch**

| Protease/Proteasekombination | Milch |
|---|---|
| M1 (V1 + V2, Verhältnis 5:1): | 7,3 |
| V1: | 6,6 |
| V2: | 6,7 |

| | |
|---|---|
| V1: Basis + 1.Protease: (Enzym gemäß Seq ID NO. 3) V2: Basis + 2. Protease (Protease, die eine Aminosäuresequenz aufweist, die in der Zählung gemäß SEQ ID NO. 2 die folgenden Aminosäuresubstitution G116V, S126L, P127Q und S128A, und ansonsten die in SEQ ID NO:2 angegebenen Aminosäuresequenz aufweist) M1: Basis + Mischung im Verhältnis 5:1 bezogen auf den Proteasegehalt in Gew.-% aus Enzym gemäß V1 und Enzym gemäß V2 | |

Tabelle 3 ist deutlich zu entnehmen, dass die erfindungsgemäße Kombination zu einer Erhöhung der Reinigungsleistung auf Milch führt.

## Patentansprüche

1. Reinigungsmittel, **dadurch gekennzeichnet, dass** es mindestens eine erste und eine zweite Protease umfasst, wobei
a) die erste Protease eine Aminosäuresequenz umfasst, die zu der in SEQ ID NO. 1 angegebenen Aminosäuresequenz über deren Gesamtlänge zu mindestens 80% identisch ist und mindestens zwei, bevorzugt mindestens drei, besonders bevorzugt vier und am meisten bevorzugt fünf der Aminosäuresubstitutionen ausgewählt aus S9R, A15T, V66A, N212D und Q239R in der Zählung gemäß SEQ ID NO. 1 aufweist, und
b) die zweite Protease eine Aminosäuresequenz umfasst, die zu der in SEQ ID NO. 2 angegebenen Aminosäuresequenz über deren Gesamtlänge zu mindestens 80% identisch ist und mindestens eine Aminosäuresubstitution an einer, zwei, drei oder vier der folgenden Positionen 116, 126, 127, 128 und 160 in der Zählung gemäß SEQ ID NO. 2 aufweist.

2. Reinigungsmittel nach Anspruch 1, **dadurch gekennzeichnet, dass** die erste Protease eine Aminosäuresequenz umfasst, die eine der folgenden Kombinationen von Aminosäuresubstitutionen aufweist: S9R+V66A+N212D+Q239R, S9R+A15T+N212D+Q239R, S9R+A15T+V66A+Q239R, S9R+A15T+V66A+N212D, A15T+V66A+N212D+Q239R; S9R+A15T+V66A, S9R+A15T+N212D, S9R+A15T+Q239R, S9R+N212D+Q239R, S9R+V66A+N212D, S9R +V66A+ Q239R, A15T+V66A+N212D, A15T+V66A+Q239R, A15T+N212D+Q239R, V66A+N212D+Q239R; S9R+A15T, S9R+V66A, S9R+N212D, S9R+ Q239R, A15T+V66A, A15T+N212D, A15T+Q239R, V66A+N212D, V66A+Q239R, N212D+Q239R.

3. Reinigungsmittel nach Anspruch 1 oder 2, **dadurch gekennzeichnet, dass** die erste Protease eine Aminosäuresequenz gemäß SEQ ID NO. 3 aufweist oder eine Aminosäuresequenz, die zu der in EQ ID NO:3 angegebenen Aminosäuresequenz mindestens zu 80% identisch ist, wobei die Positionen 9, 15, 66, 212 und 239 invariabel sind.

4. Reinigungsmittel nach einem der Ansprüche 1 bis 3, **dadurch gekennzeichnet, dass** die zweite Protease eine Aminosäuresequenz aufweist, die zu der in SEQ ID NO. 2 angegebenen Aminosäuresequenz über deren Gesamtlänge zu mindestens 80%, bevorzugt zu mindestens 90 %, insbesondere zu 100 % identisch ist und die in der Zählung gemäß SEQ ID NO. 2 mindestens eine, bevorzugt mehrere, insbesondere jede der folgenden Aminosäuresubstitution G116V, S126L, P127Q und/oder S128A aufweist.

5. Reinigungsmittel nach einem der Ansprüche 1 bis 4, **dadurch gekennzeichnet, dass** es eine erste Protease und eine zweite Protease in einem Massenverhältnis von 10:1 bis 1:10 enthält.

6. Reinigungsmittel nach einem der Ansprüche 1 bis 5, **dadurch gekennzeichnet, dass** der Gewichtsanteil jeder der Proteasen, bezogen auf das entsprechende aktive Protein, am Gesamtgewicht des Mittels von 1 x 10⁻⁸ bis 5 Gew.-%, bevorzugt 0,001 bis 2 Gew.-%, weiter bevorzugt von 0,005 bis 1,5 Gew.-%, noch weiter bevorzugt von 0,01 bis 1 Gew.-% und besonders bevorzugt von 0,01 bis 0,5 Gew.-%, beträgt.

7. Reinigungsmittel nach einem der Ansprüche 1 bis 6, **dadurch gekennzeichnet, dass** es mindestens einen weiteren Bestandteil, vorzugsweise mindestens zwei weitere Bestandteile, ausgewählt aus der Gruppe bestehend aus Gerüststoffen, Tensiden, Polymeren, Bleichmitteln, Bleichkatalysatoren, Bleichaktivatoren, Nicht-Protease-Enzymen, Korrosionsinhibitoren, Glaskorrosionsinhibitoren, Desintegrationshilfsmitteln, Duftstoffen und Parfümträgern enthält.

8. Reinigungsmittel nach einem der Ansprüche 1, bis 7, **dadurch gekennzeichnet, dass** es ein Geschirrspülmittel, insbesondere ein maschinelles Geschirrspülmittel ist.

9. Reinigungsmittel nach Anspruch 8, **dadurch gekennzeichnet, dass** das
(1) maschinelle Geschirrspülmittel in fester Form vorliegt; und/oder
(2) maschinelle Geschirrspülmittel in vorportionierter Form vorliegt; und/oder
(3) maschinelle Geschirrspülmittel mehrere räumlich voneinander getrennte Zusammensetzungen aufweist.

10. Verwendung eines Reinigungsmittels nach einem der Ansprüche 1 bis 9 in einem maschinellen Geschirrspülverfahren.

11. Verfahren zur Reinigung von Geschirr in einer automatischen Geschirrspülmaschine, **dadurch gekennzeichnet, dass** ein Reinigungsmittel nach einem der Ansprüche 1 bis 9 während des Durchlaufens eines Geschirrspülprogramms vor Beginn des Hauptspülgangs oder im Verlaufe des Hauptspülgangs in den Innenraum einer Geschirrspülmaschine eindosiert wird.

12. Verwendung einer Kombination einer ersten und einer zweiten Protease zur Verbesserung der Reinigungsleistung, insbesondere der Reinigungsleistung an enzymsensitiven Anschmutzungen, eines Reinigungsmittels, vorzugsweise eines maschinellen Geschirrspülmittels, **dadurch gekennzeichnet, dass**
a) die erste Protease eine Aminosäuresequenz umfasst, die zu der in SEQ ID NO. 1 angegebenen Aminosäuresequenz über deren Gesamtlänge zu mindestens 80% identisch ist und mindestens zwei, bevorzugt mindestens drei, besonders bevorzugt vier und am meisten bevorzugt fünf der Aminosäuresubstitutionen ausgewählt aus S9R, A15T, V66A, N212D und Q239R in der Zählung gemäß SEQ ID NO. 1 aufweist, und
b) die zweite Protease eine Aminosäuresequenz umfasst, die zu der in SEQ ID NO. 2 angegebenen Aminosäuresequenz über deren Gesamtlänge zu mindestens 80 % identisch ist und mindestens eine Aminosäuresubstitution an einer, zwei, drei oder vier der folgenden Positionen 116, 126, 127, 128 und 160 in der Zählung gemäß SEQ ID NO. 2 aufweist.

## Claims

1. A cleaning agent, **characterized in that** it comprises at least a first protease and a second protease,
a) the first protease comprising an amino acid sequence which is at least 80% identical to the amino acid sequence specified in SEQ ID NO. 1 over the total length thereof and has at least two, preferably at least three, particularly preferably four, and most preferably five of the amino acid substitutions selected from S9R, A15T, V66A, N212D and Q239R using the numbering according to SEQ ID NO. 1, and
b) the second protease comprises an amino acid sequence which is at least 80% identical to the amino acid sequence specified in SEQ ID NO. 2 over the total length thereof and has at least one amino acid substitution at one, two, three or four of the following positions 116, 126, 127, 128 and 160 using the numbering according to SEQ ID NO. 2.

2. The cleaning agent according to claim 1, **characterized in that** the first protease comprises an amino acid sequence having one of the following combinations of amino acid substitutions: S9R+V66A+N212D+Q239R, S9R+A15T+N212D+Q239R, S9R+A15T+V66A+Q239R, S9R+A15T+V66A+N212D, A15T+V66A+N212D+Q239R; S9R+A15T+V66A, S9R+A15T+N212D, S9R+A15T+Q239R, S9R+N212D+Q239R, S9R+V66A+N212D, S9R+V66A+Q239R, A15T+V66A+N212D, A15T+V66A+Q239R, A15T+N212D+Q239R, V66A+N212D+Q239R; S9R+A15T, S9R+V66A, S9R+N212D, S9R+ Q239R, A15T+V66A, A15T+N212D, A15T+Q239R, V66A+N212D, V66A+Q239R, N212D+Q239R.

3. The cleaning agent according to claim 1 or 2, **characterized in that** the first protease has an amino acid sequence according to SEQ ID NO. 3 or an amino acid sequence which is at least 80% identical to the amino acid sequence specified in SEQ ID NO. 3, the positions 9, 15, 66, 212 and 239 being invariable.

4. The cleaning agent according to one of claims 1 to 3, **characterized in that** the second protease has an amino acid sequence which is at least 80%, preferably at least 90%, in particular 100% identical to the amino acid sequence specified in SEQ ID NO. 2 over the total length thereof, and which, in the numbering according to SEQ ID NO. 2, has at least one, preferably a plurality of, in particular each of the following amino acid substitutions G116V, S126L, P127Q and/or S128A.

5. The cleaning agent according to one of claims 1 to 4, **characterized in that** it contains a first protease and a second protease in a mass ratio of from 10:1 to 1:10.

6. The cleaning agent according to one of claims 1 to 5, **characterized in that** the weight fraction of each of the proteases, based on the corresponding active protein, with respect to the total weight of the agent is from 1 x 10⁻⁸ to 5 wt. %, preferably 0.001 to 2 wt.%, more preferably 0.005 to 1.5 wt.%, even more preferably 0.01 to 1 wt.% and particularly preferably 0.01 to 0.5 wt.%.

7. The cleaning agent according to one of claims 1 to 6, **characterized in that** it contains at least one further component, preferably at least two further components, selected from the group consisting of builders, surfactants, polymers, bleaching agents, bleach catalysts, bleach activators, non-protease enzymes, corrosion inhibitors, glass corrosion inhibitors, disintegration auxiliaries, fragrances and perfume carriers.

8. The cleaning agent according to one of claims 1 to 7, **characterized in that** it is a dishwashing detergent, in particular an automatic dishwashing detergent.

9. The cleaning agent according to claim 8, **characterized in that**
(1) the automatic dishwashing detergent is in solid form; and/or
(2) the automatic dishwashing detergent is in a pre-portioned form; and/or
(3) the automatic dishwashing detergent has a plurality of compositions that are spatially separated from one another.

10. The use of a cleaning agent according to one of claims 1 to 9 in an automatic dishwashing method.

11. A method for cleaning dishes in an automatic dishwasher, **characterized in that** a cleaning agent according to one of claims 1 to 9 is dispensed into the interior of a dishwasher while a dishwashing program is running, before the main washing cycle begins or during the main washing cycle.

12. The use of a combination of a first and a second protease for improving cleaning performance, in particular the cleaning performance with respect to enzyme-sensitive stains, of a cleaning agent, preferably an automatic dishwashing detergent, **characterized in that**
a) the first protease comprises an amino acid sequence which is at least 80% identical to the amino acid sequence specified in SEQ ID NO. 1 over the total length thereof and has at least two, preferably at least three, particularly preferably four, and most preferably five of the amino acid substitutions selected from S9R, A15T, V66A, N212D and Q239R using the numbering according to SEQ ID NO. 1, and
b) the second protease comprises an amino acid sequence which is at least 80% identical to the amino acid sequence specified in SEQ ID NO. 2 over the total length thereof and has at least one amino acid substitution at one, two, three or four of the following positions 116, 126, 127, 128 and 160 using the numbering according to SEQ ID NO. 2.

## Revendications

1. Détergent, **caractérisé en ce qu'**il comprend au moins une première et une deuxième protéase,
a) la première protéase comprenant une séquence d'acides aminés qui est identique à au moins 80 % sur toute sa longueur à la séquence d'acides aminés indiquée en SEQ ID NO. 1 et présentant au moins deux, de préférence au moins trois, de manière particulièrement préférée quatre et de manière préférée entre toutes cinq, des substitutions d'acides aminés choisies parmi S9R, A15T, V66A, N212D et Q239R dans le comptage selon SEQ ID NO. 1, et
b) la deuxième protéase comprenant une séquence d'acides aminés qui est identique à au moins 80 % sur toute sa longueur à la séquence d'acides aminés indiquée en SEQ ID NO. 2 et présentant au moins une substitution d'acide aminé à une, deux, trois ou quatre des positions suivantes 116, 126, 127, 128 et 160 dans le comptage selon SEQ ID NO. 2.

2. Détergent selon la revendication 1, **caractérisé en ce que** la première protéase comprend une séquence d'acides aminés, présentant une des combinaisons suivantes de substitutions d'acides aminés : S9R+V66A+N212D+Q239R, S9R+A15T+N212D+Q239R, S9R+A15T+V66A+Q239R, S9R+A15T+V66A+N212D, A15T+V66A+N212D+Q239R; S9R+A15T+V66A, S9R+A15T+N212D, S9R+A15T+Q239R, S9R+N212D+Q239R, S9R+V66A+N212D, S9R+V66A+Q239R, A15T+V66A+N212D, A15T+V66A+Q239R, A15T+N212D+Q239R, V66A+N212D+Q239R; S9R+A15T, S9R+V66A, S9R+N212D, S9R+Q239R, A15T+V66A, A15T+N212D, A15T+Q239R, V66A+N212D, V66A+Q239R, N212D+Q239R.

3. Détergent selon la revendication 1 ou 2, **caractérisé en ce que** la première protéase comporte une séquence d'acides aminés selon SEQ ID NO. 3 ou une séquence d'acides aminés qui est identique à au moins 80 % à la séquence d'acides aminés indiquée en SEQ ID NO. 3, les positions 9, 15, 66, 212 et 239 étant invariables.

4. Détergent selon l'une des revendications 1 à 3, **caractérisé en ce que** la deuxième protéase comporte une séquence d'acides aminés qui est identique à au moins 80 % sur toute sa longueur à la séquence d'acides aminés indiquée en SEQ ID NO. 2, de préférence à au moins 90 %, en particulier à 100 %, et présente au moins une substitution d'acide aminé, de préférence plusieurs, en particulier chacune des substitutions d'acides aminés suivantes G116V, S126L, P127Q et/ou S128A dans le comptage selon SEQ ID NO.2.

5. Détergent selon l'une des revendications 1 à 4, **caractérisé en ce qu'**il contient une première protéase et une deuxième protéase selon un rapport massique de 10 :1 à 1 :10.

6. Détergent selon l'une des revendications 1 à 5, **caractérisé en ce que** la proportion en poids de chacune des protéases représente, à l'égard de la protéine active correspondante, et par rapport au poids total de l'agent, de 1 x 10⁻⁸ à 5 % en poids, de préférence de 0,001 à 2 % en poids, de manière davantage préférée de 0,005 à 1,5 % en poids, de manière encore davantage préférée de 0,01 à 1 % en poids et de manière particulièrement préférée de 0,01 à 0,5 % en poids.

7. Détergent selon l'une des revendications 1 à 6, **caractérisé en ce qu'**il contient au moins un autre constituant, de préférence au moins deux autres constituants, choisis dans le groupe constitué d'adjuvants, de tensioactifs, de polymères, d'agents de blanchiment, de catalyseurs de blanchiment, d'activateurs de blanchiment, d'enzymes autres que la protéase, d'inhibiteurs de la corrosion, d'inhibiteurs de la corrosion du verre, d'adjuvants de désintégration, de substances odoriférantes et de supports de parfum.

8. Détergent selon l'une des revendications 1 à 7, **caractérisé en ce qu'**il s'agit d'un détergent pour laver la vaisselle, en particulier d'un détergent pour laver la vaisselle en machine.

9. Détergent selon la revendication 8, **caractérisé en ce que**
(1) le détergent pour laver la vaisselle en machine se présente sous forme solide ; et/ou
(2) le détergent pour laver la vaisselle en machine se présente sous forme de portions préétablies ; et/ou
(3) le détergent pour laver la vaisselle en machine contient plusieurs compositions séparées spatialement les unes des autres.

10. Utilisation d'un détergent selon l'une des revendications 1 à 9 dans un procédé de lavage de la vaisselle en machine.

11. Procédé de lavage de la vaisselle dans un lave-vaisselle automatique, **caractérisé en ce qu'**un détergent selon l'une des revendications 1 à 9 est introduit dans l'espace interne d'un lave-vaisselle pendant l'exécution d'un programme de lavage de la vaisselle avant le début du cycle de lavage principal ou au cours du cycle de lavage principal.

12. Utilisation d'une combinaison d'une première et d'une deuxième protéase pour améliorer l'efficacité de lavage, en particulier l'efficacité de lavage à l'égard de saletés sensibles aux enzymes, d'un détergent, de préférence d'un détergent pour laver la vaisselle en machine, **caractérisée en ce que**
a) la première protéase comprend une séquence d'acides aminés qui est identique à au moins 80 % sur toute sa longueur à la séquence d'acides aminés indiquée en SEQ ID NO. 1 et présentant au moins deux, de préférence au moins trois, de manière particulièrement préférée quatre et de manière préférée entre toutes cinq, des substitutions d'acides aminés choisies parmi S9R, A15T, V66A, N212D et Q239R dans le comptage selon SEQ ID NO. 1, et
b) la deuxième protéase comprend une séquence d'acides aminés qui est identique à au moins 80 % sur toute sa longueur à la séquence d'acides aminés indiquée en SEQ ID NO. 2 et présentant au moins une substitution d'acides aminés à une, deux, trois ou quatre des positions suivantes 116, 126, 127, 128 et 160 dans le comptage selon SEQ ID NO. 2.
